(19) Europäisches Patentamt
European Patent Office
Office européen des brevets

(11) Veröffentlichungsnummer: **0 154 886**
**A2**

(12) # EUROPÄISCHE PATENTANMELDUNG

(21) Anmeldenummer: **85102134.5**

(22) Anmeldetag: **27.02.85**

(51) Int. Cl.[4]: **C 07 K 5/06**, A 61 K 37/02

(30) Priorität: **12.03.84 DE 3408923**

(43) Veröffentlichungstag der Anmeldung: **18.09.85 Patentblatt 85/38**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Henning, Rainer, Dr., Rotenhofstrasse 31, D-6234 Hattersheim am Main (DE)**
Erfinder: **Urbach, Hansjörg, Dr., Le Lavandoustrasse 41, D-6242 Kronberg/Taunus (DE)**
Erfinder: **Becker, Reinhard, Dr., Adelheidstrasse 101, D-6200 Wiesbaden (DE)**
Erfinder: **Teetz, Volker, Dr., An der Tann 20, D-6238 Hofheim am Taunus (DE)**

(54) **Carboxyalkyldipeptidderivate, Verfahren zu deren Herstellung, diese enthaltende Mittel und deren Verwendung.**

(57) Die Erfindung betrifft Verbindungen der Formel I,

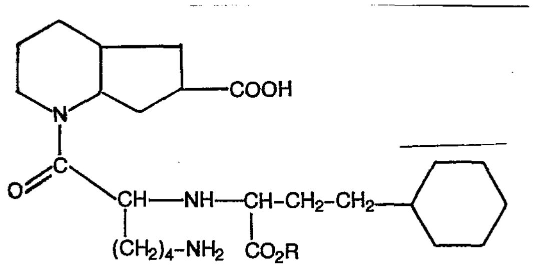

in welcher R Wasserstoff oder ($C_1$–$C_6$)-Alkyl bedeutet, sowie deren Salze, Verfahren zur Herstellung dieser Verbindungen, diese enthaltende Mittel und deren Verwendung.

EP 0 154 886 A2

HOECHST AKTIENGESELLSCHAFT HOE 84/F 047 Dr.WS/mü

# Carboxyalkyldipeptidderivate, Verfahren zu deren Herstellung, diese enthaltende Mittel und deren Verwendung

In der europäischen Patentanmeldung EP-A-46953 werden Carboxyalkyldipeptidderivate als Hemmstoffe des Angiotensin-Converting-Enzyme beschrieben. Es wurde nun überraschenderweise gefunden, daß bestimmte Verbindungen aus der Reihe der dort allgemein beschriebenen Verbindungen besonders vorteilhafte Eigenschaften aufweisen.

Die Erfindung betrifft daher Verbindungen der Formel I,

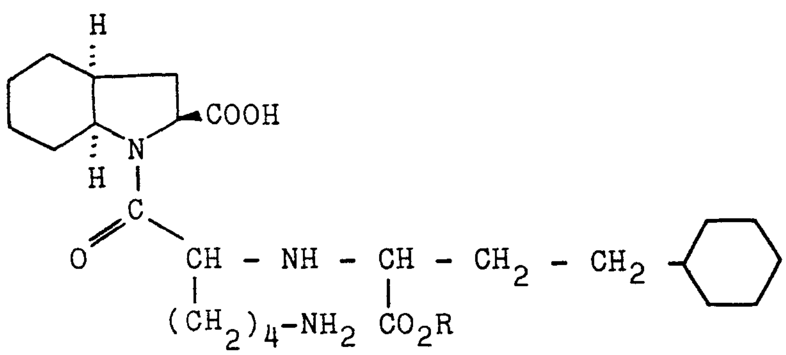

(I)

in welcher R Wasserstoff oder $(C_1-C_6)$-Alkyl bedeutet und deren physiologisch verträglichen Salze.

Bevorzugt ist R= Wasserstoff oder Ethyl.

Als Salze kommen insbesondere Alkali- und Erdalkalisalze, Salze mit physiologisch verträglichen Aminen und Salze mit anorganischen oder organischen Säuren wie beispielsweise Salzsäure, Bromwasserstoffsäure, Schwefelsäure, Maleinsäure oder Fumarsäure in Betracht.

Die erfindungsgemäßen Verbindungen weisen an allen fünf im Molekül vorkommenden Asymmetriezentren vorzugsweise die S-Konfiguration auf.

Die Erfindung betrifft weiterhin ein Verfahren zur Herstellung von Verbindungen der Formel I, das dadurch gekennzeichnet ist, daß man

a) Verbindungen der Formel II,

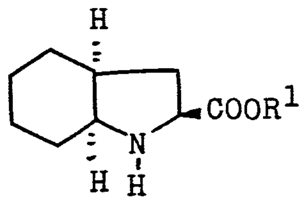

(II)

in welcher $R^1$ einen hydrogenolytisch oder hydrolytisch leicht abspaltbaren Rest, wie beispielsweise eine Benzyl- oder eine tert. Butylgruppe bedeutet, mit Verbindungen der Formel III,

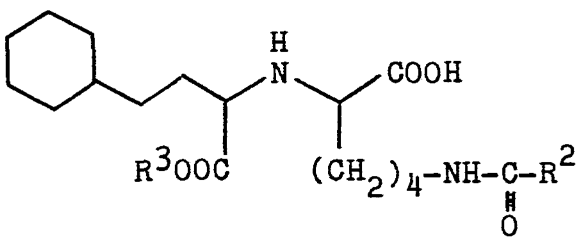

(III)

in welcher $R^2$ eine Urethan-Schutzgruppe bedeutet und vorzugsweise wie $OR^1$ definiert ist und dabei gleich wie $OR^1$ oder davon verschieden sein kann, und in welcher $R^3$ Benzyl oder $(C_1-C_6)$-Alkylrest wie z.B. Ethyl oder tert. Butyl bedeutet, nach einer der üblichen Methoden der Peptidchemie umsetzt, beispielsweise mittels Dicyclohexylcarbodiimid/N-Hydroxy-benztriazol oder N-Propylphosphonsäureanhydrid, oder

b) Verbindungen der Formel IV,

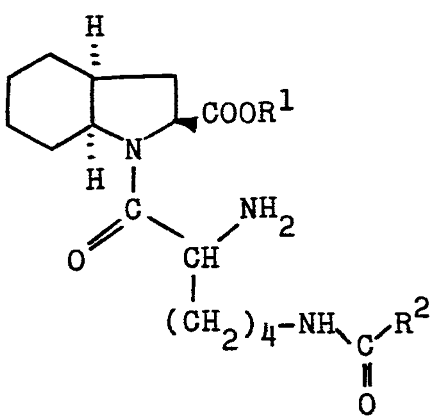

(IV)

in welcher $R^1$ und $R^2$ wie oben bei Formeln II und III definiert sind, mit Verbindungen der Formel V,

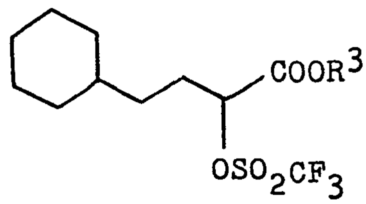

(V)

in welcher $R^3$ wie oben bei Formel III definiert ist, in Gegenwart einer Base wie Triethylamin, umsetzt,

aus gegebenenfalls gebildeten Isomerengemischen die gewünschten Isomeren, vorzugsweise diejenigen Verbindungen mit all-S-Konfiguration abtrennt, die Schutzgruppen $R^1$ und $R^2$ abspaltet und $R^3$ gegebenenfalls abspaltet, was in einem Schritt oder in mehreren Schritten erfolgen kann, erhaltene Verbindungen der Formel I gegebenenfalls verestert und gegebenenfalls in physiologisch verträgliche Salze überführt, wobei bei einer Schutzgruppenabspaltung in mehreren Schritten die Veresterung auch auf einer früheren Verfahrensstufe erfolgen kann.

Die Umsetzung einer Verbindung der Formel II mit einer Verbindung der Formel III zur Herstellung einer Verbindung der Formel I erfolgt gemäß einer in der Peptidchemie bekannten Kondensationsreaktion, wobei als Kondensationsmittel beispielsweise Dicyclohexylcarbodiimid und 1-Hydroxybenztriazol (inertes Lösungsmittel wie DMF, Zusatz einer Base wie N-Ethylmorpholin, Reaktionstemperatur zwischen -20°C und dem Siedepunkt des Lösungsmittels, vorzugsweise 0 bis +50°C) oder N-Alkylphosphonsäureanhydrid (inertes Lösungsmittel wie Methylenchlorid, gegebenenfalls Zusatz einer Base wie N-Ethylmorpholin, Reaktionstemperatur -10 bis +50°C, vorzugsweise zwischen 0°C und Raumtemperatur) zugesetzt werden.

Die Umsetzung einer Verbindung der Formel IV mit einer Verbindung der Formel V zur Herstellung einer Verbindung

der Formel I erfolgt zweckmäßig in einem inerten Lösungsmittel wie Methylenchlorid in Gegenwart eines Säurefängers wie Triethylamin oder $Na_2CO_3$ zwischen -80°C und +150°C, vorzugsweise zwischen -20°C und +80°C.

Bei der nachfolgenden hydrogenolytischen Abspaltung der Schutzgruppe(n) wird als Katalysator bevorzugt Palladium verwendet, während bei der sauren Abspaltung der Schutzgruppe(n) als Säuren bevorzugt Trifluoressigsäure oder Chlorwasserstoff eingesetzt werden.

Die Veresterung zur Herstellung von Verbindungen der Formel I mit $R \neq$ Wasserstoff kann beispielsweise durch Umsetzung der entsprechenden Carbonsäure mit einem Überschuß eines Alkohols in Gegenwart eines sauren Katalysators (z.B. einer Mineralsäure, wie $H_2SO_4$, einer Arylsulfonsäure oder eines sauren Kationenaustauschers) zwischen 0°C und dem Siedepunkt des Reaktionsgemisches erfolgen.

Verbindungen der Formel II sind beispielsweise aus der EP-A-46953 bekannt. Verbindungen der Formel III erhält man, indem man 3-Cyclohexylpropanol zum entsprechenden Aldehyd oxydiert, vorzugsweise mittels Oxalylchlorid/Dimethylsulfoxid/Triethylamin (Synthesis <u>1981</u>, 165), diesen über das Bisulfitaddukt in das Cyanhydrin überführt und zur Hydroxysäure der Formel VI ($R^3$= H) verseift;

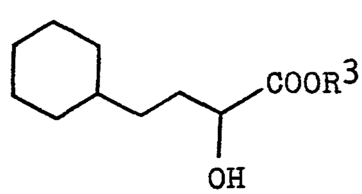

(VI)

Veresterung mit einem entsprechenden Alkohol führt zu den Estern der Formel VI ($R^3 \neq$ H), deren Umsetzung mit Trifluormethansulfonsäureanhydrid in Gegenwart von Pyridin die Triflate der Formel V ergibt. Aus diesen werden

0154886

mit einem geeignet geschützten Lysin-Derivat, vorzugsweise dem $N^{\varepsilon}$-Benzyloxycarbonyl-tert.butylester und nachfolgender Abspaltung der Estergruppe, vorzugsweise mittels Trifluoressigsäure die Verbindungen der Formel III erhalten. Verfahren zur Herstellung von Verbindungen der Formel IV sind beispielsweise aus der EP-A-46953 bekannt. Es sind solche Verbindungen der Formeln II bzw. IV bevorzugt, in welchen das C-Atom 2 des bicyclischen Ringsystems S-konfiguriert ist und in welchen dieses Ringsystem in der cis,endo-Konfiguration vorliegt.

Die erfindungsgemäßen Verbindungen der Formel I liegen als inneres Salz vor. Als amphotere Verbindungen können sie Salze mit Säuren und Basen bilden. Diese Salze werden in üblicher Weise durch Umsetzung mit einem oder zwei Äquivalenten Säure bzw. Base hergestellt.

Die erfindungsgemäßen Verbindungen der Formel I sind Hemmer des Angiotensin-Converting-Enzyme (ACE), das die Umwandlung von Angiotensin I in das pressorisch wirksame Angiotensin II katalysiert. Z.B. hemmt die Verbindung der Formel I mit R= Wasserstoff beim in vitro-Assay (Biochem. Biophys. Acta, 206, 136 (1970)) das ACE mit einer $IC_{50}$ von $3{,}4 \cdot 10^{-9}$ M. Nach intraduodenaler Gabe an der narkotisierten Ratte wird eine starke Hemmwirkung auf die durch intravenöse Gabe von 310 ng Angiotensin hervorgerufene Pressorreaktion beobachtet. Besonders interessant ist hierbei der langsame Wirkungseintritt und die lange Wirkdauer der Verbindung. So beobachtet man bei intraduodenaler Gabe von 0,3 mg/kg Körpergewicht eine Hemmung um 15 % nach 10 min, 30 % nach 20 min, 60 % nach 30 min und 70 % nach 45 min; die Wirkung hält länger als 6 Stunden an.

Die Verbindungen der Formel I und ihre Salze besitzen lang anhaltende, intensiv blutdrucksenkende Wirkung. Sie können zur Bekämpfung des Bluthochdrucks verschiedener

Genese eingesetzt werden. Auch ihre Kombination mit anderen blutdrucksenkenden, gefäßerweiternden oder diuretisch wirkenden Verbindungen ist möglich. Typische Vertreter dieser Wirkstoffklassen sind z.B. in Erhardt-Ruschig, Arzneimittel, 2. Auflage, Weinheim 1972 beschrieben. Die Anwendung kann intravenös, subcutan oder peroral erfolgen.

Die Dosierung liegt bei peroraler Gabe bei 0,01 - 7 mg/kg/Tag, insbesondere bei 0,07 - 0,5 mg/kg/Tag. Sie kann in schweren Fällen auch erhöht werden, da toxische Effekte bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsform gebracht, wie Tabletten, Dragees, Steckkapseln, wäßrige,alkoholische oder ölige Suspensionen oder wäßrige, alkoholische oder ölige Lösungen. Als inerte Träger kommen z.B. Gummi arabicum, Magnesiumcarbonat, Kaliumphosphat, Milchzucker, Glucose oder Stärke, insbesondere Maisstärke in Betracht. Dabei kann die Zubereitung sowohl als Trocken- als auch als Feuchtgranulat erfolgen. Als ölige Trägerstoffe oder Lösungsmittel kommen beispielsweise pflanzliche oder tierische Öle in Betracht, wie Sonnenblumenöl oder Lebertran.

Zur subcutanen oder intravenösen Applikation werden die aktiven Verbindungen oder deren physiologisch verträglichen Salze, gewünschtenfalls mit den dafür üblichen Substanzen wie Lösungsvermittler oder weiteren Hilfsstoffen in Lösung, Suspension oder Emulsion gebracht.

Als Lösungsmittel für die neuen aktiven Verbindungen und deren Salze kommen z.B. in Frage: Wasser, Propandiol oder Glycerin, daneben auch Zuckerlösungen wie Glucose- oder Mannitlösungen oder auch eine Mischung aus den genannten Lösungsmitteln.

Die nachfolgenden Beispiele dienen der Erläuterung der Erfindung.

Beispiel 1

N-(1-S-Carboxy-3-cyclohexyl-propyl)-S-lysyl-2S,3aS,7aS-octahydroindol-2-carbonsäure

a) 3-Cyclohexyl-propanal

14,8 ml frisch destilliertes Oxalylchlorid werden in 370 ml Methylenchlorid gelöst und auf -78°C gekühlt. 25,2 ml (0,33 Mol) Dimethylsulfoxid in 740 ml Methylenchlorid werden zugetropft und nach 5 Minuten 21 g (0,148 Mol) 3-Cyclohexylpropanol in 150 ml Methylenchlorid hinzugefügt. Nach weiteren 30 min tropft man 103,6 ml Triethylamin zu, rührt noch 10 min nach und wärmt dann auf Raumtemperatur auf. Nach Wasserzusatz werden die Phasen getrennt, die wäßrige Phase nochmals mit Methylenchlorid extrahiert, die vereinigten organischen Phasen mit gesättigter Kochsalz-Lösung gewaschen, über Natriumsulfat getrocknet und eingeengt. Das Rohprodukt wird über Kieselgel mit Essigester/Cyclohexan (1 : 2) als Laufmittel filtriert. Man erhält 19,7 g farbloses Öl.

$^1$H-NMR ($CDCl_3$) : $\delta$= 9,55 (br. s, 1H);
2,37 (t, 2H);
2,0 - 0,8 (m, 13H) ppm.

b) 4-Cyclohexyl-2-hydroxy-butyronitril

19,7 g (0,15 Mol) 3-Cyclohexylpropanal werden unter Rühren mit gesättigter Natriumbisulfitlösung versetzt, bis sich kein Niederschlag mehr bildet (ca. 100 ml). Nach 10 min wird mit 50 ml Ethanol verdünnt und 30 min gerührt, der Niederschlag abgesaugt und mit Ethanol und Ether gewaschen. Das rohe Bisulfit-Addukt (43,25 g) wird in 600 ml Wasser bei 40°C gelöst, 7,8 g (0,12 Mol) Kaliumcyanid in 30 ml Wasser werden zugesetzt. Nach 45 min wird mit Ether extrahiert, mit Natriumsulfat getrocknet und eingeengt. Das Rohprodukt wird direkt weiterverwendet.

c) 4-Cyclohexyl-2-hydroxy-buttersäure

Das Nitril aus Beispiel 1b) wird mit 100 ml konzentrierter Salzsäure 2,5 Stunden am Rückfluß erhitzt, die Lösung wird mit 300 ml Wasser verdünnt, mit Ether extrahiert, mit Natriumsulfat getrocknet und eingeengt. Der wachsartige Rückstand wird mit Petrolether verrieben und abgesaugt. Man erhält 6,9 g Produkt.

$^{1}H$-NMR ($CDCl_3$) $\delta$ = 4,26 (t, 1H)
2,1 - 0,8 (m, 15H) ppm.

d) 4-Cyclohexyl-2-hydroxy-buttersäureethylester

6,9 g (0,037 Mol) 4-Cyclohexyl-2-hydroxybuttersäure werden in 300 ml absolutem Ethanol gelöst. Nach Zugabe von 0,3 ml konz. Schwefelsäure wird 3,5 Stunden am Rückfluß erhitzt, dann eingeengt, in Ether aufgenommen, je 1 mal mit Wasser und 10 %iger Natriumcarbonat-Lösung gewaschen, über Natriumsulfat getrocknet und eingeengt; 7,7 g farbloses Öl.

$^1$H-NMR ($CDCl_3$) : $\delta$ = 4,23 (q, 2H);
4,17 (t, 1H);
2,45 (br. s, 1H);
2,0 - 0,9 (m, 1 5H);
1,3 (t, 3H) ppm

e) 4-Cyclohexyl-2-trifluormethansulfonyloxy-buttersäure-ethylester

Zu einer Lösung von 0,6 ml Pyridin (75 mmol) und 1,45 g (7,1 mmol) 4-Cyclohexyl-2-hydroxybuttersäureethylester in 40 ml trockenem Methylenchlorid werden bei -10°C 2,1 g (1,25 ml, 7,5 mmol) Trifluormethansulfonsäureanhydrid getropft. Nach 10 min wird auf Raumtemperatur erwärmt und 30 min nachgerührt. Nach Einengen wird durch wenig Kieselgel mit n-Hexan als Laufmittel filtriert. Man erhält 2 g farbloses Öl.

$^1$H-NMR ($CDCl_3$) : $\delta$ = 5,07 (t, 1H);
4,30 (q, 2H);
2,2 - 0,9 (m, 15H);
1,32 (t, 3H) ppm.

f) $N^{\alpha}$(3-Cyclohexyl-1-ethoxycarbonyl)-propyl-$N^{\varepsilon}$-benzyloxycarbonyl-S-lysin-tert.butylester

4,84 g (14,4 mmol) N-Benzyloxycarbonyl-S-lysin-tert. butylester und 1,99 ml Triethylamin werden in 50 ml absolutem Methylenchlorid gelöst. Bei Raumtemperatur tropft man eine Lösung von 4 g 4-Cyclohexyl-2-trifluormethansulfonyloxy-buttersäureethyleester in 20 ml Methylenchlorid zu. Nach 20 Stunden Rühren bei Raumtemperatur wird mit Wasser gewaschen, mit Natriumsulfat getrocknet und eingeengt. Das Rohprodukt (9,3 g) wird an 220 g Kieselgel mit Essigester/Cyclohexan (1 : 2) als Laufmittel chromatographiert, wodurch die zwei Isomeren getrennt werden:

Isomer 1 (R,S-Konfiguration): 3,6 g; $[\alpha]_D^{20} = -1,75°$ (c= 0,4; Methanol)

$^1H$-NMR ($CDCl_3$) : $\delta$ = 7,34 (s, 5H);
5,10 (s, 2H);
5,0 - 4,6 (m, 1H);
4,17 (q, 2H);
3,4 - 3,0 (m, 4H);
2,0 - 0,9 (m, 18H);
1,43 (s, 9H);
1,27 (t, 3H) ppm.

Isomer 2 (S,S-Konfiguration): 3,8 g; $[\alpha]_D^{20} = -10,5°$ (c= 0,4; Methanol)

$^1H$-NMR ($CDCl_3$) : $\delta$ = 7,33 (s, 5H);
5,07 (s, 2H);
5,1 - 4,7 (m, 1H);
4,17 (q,2H);
3,4 - 3,0 (m, 4H);
1,46 (s, 9H);
1,27 (t, 3H);
2,0 - 0,9 (m, 18H) ppm.

g) $N^\alpha$-(3-Cyclohexyl-1-S-ethoxycarbonyl)-propyl-$N^\varepsilon$-benzyloxycarbonyl-S-lysin

3,8 g des Isomers 2 aus Beispiel 1 f) werden in 20 ml Trifluoressigsäure 2 Stunden gerührt. Die Säure wird im Vakuum entfernt, der Rückstand dreimal mit Toluol eingeengt. Der Rückstand wird in Wasser/Methanol aufgenommen und unter Rühren mit Amberlite® IRA 93 (Acetatform) bis zu einem pH-Wert von 4,2 versetzt, filtriert und eingeengt. Das Rohprodukt wird an 30 g Kieselgel mit Methanol/Methylenchlorid (1 : 4) als Laufmittel gereinigt. Man erhält 2,7 g farbloses Harz.

$^1$H-NMR ($CDCl_3$) : $\delta$ = 7,3 (s, 5H);
6,1 (br.s, 3H);
5,07 (s, 2H);
4,25 (q, 2H);
3,5 - 3,0 (m, 4H);
2,0 - 0,9 (m, 18H);
1,27 (t, 3H) ppm.

h) $N^\alpha$-(3-Cyclohexyl-1-S-ethoxycarbonyl)-propyl-N-benzyloxycarbonyl-S-lysyl-2S,3aS,7aS-octahydroindol-2-carbonsäurebenzylester

Aus 1,52 g 2S, 3aS, 7aS-Octahydroindol-2-carbonsäurebenzylester. Hydrochlorid wird mit NaOH die Base freigesetzt und mit 1,35 g der Verbindung aus Beispiel 1 g), 0,34 g 1-Hydroxybenztriazol und 0,38 ml N-Ethylmorpholin in 20 ml DMF gelöst. Bei 0°C werden 0,63 g Dicyclohexylcarbodiimid zugesetzt, 2,5 Std. bei Raumtemperatur gerührt, mit Essigester verdünnt, filtriert und das Filtrat je 1 mal mit 10 %iger Citronensäure, 1N Natriumbicarbonatlösung, Wasser und gesättigter Kochsalzlösung gewaschen, über Natriumsulfat getrocknet und eingeengt. Chromatographie an Kieselgel mit Essigester/Cyclohexan (1 : 2) als Laufmittel ergibt 1,3 g Produkt als farbloses Öl.

$^1$H-NMR ($CDCl_3$) : $\delta$ = 7,3 (s, 10H);
5,06 (s, 4H);
4,18 (q, 2H);
5,0 - 3,6 (m, 4H);
3,6 - 3,0 (m, 4H);
2,5 - 0,9 (m, 32H);
1,27 (t, 3H) ppm.

i) $N^\alpha$-(3-Cyclohexyl-1-S-ethoxycarbonyl)-propyl-S-lysyl-2S,3aS,7aS-octahydroindol-2-carbonsäure

1,3 g der Verbindung aus Beispiel 1 h) werden in 50 ml

Ethanol mit 0,4 g Palladium auf Kohle (10 %) bei 1 bar Wasserstoff-Druck hydriert (ca. 1 Std.). Nach Abfiltrieren des Katalysators wird eingeengt. Man erhält 750 mg farblosen Schaum.

$^1$H-NMR ($CDCl_3$) : $\delta$ = 4,2 (q, 2H);
5,0 - 2,6 (m, 8H);
2,5 - 0,9 (m, 32H);
1,25 (t, 3H) ppm.

j) $N^\alpha$-(1-S-Carboxy-3-cyclohexyl)-propyl-S-lysyl-2S,3aS, 7aS-octahydroindol-2-carbonsäure

410 mg (0,83 mmol) der Verbindung aus Beispiel 1 i) werden in 5 ml Wasser gelöst, 0,62 ml 4 N KOH werden zugesetzt und 5 Stunden bei Raumtemperatur gerührt. Mit konzentrierter HCl wird auf einen pH-Wert von 5 gestellt, die Lösung auf eine Amberlite® IR 120-Säule ($H^+$-Form) gegeben, mit Wasser neutral gewaschen und mit 2 % Pyridin in Wasser eluiert. Das Lösungsmittel wird entfernt, der Rückstand dreimal mit Toluol eingeengt und mit Methylenchlorid/Isopropylether verrieben. Man erhält 285 mg Produkt vom Schmp. 171°C (Zersetzung).

Beispiel 2

$N^\alpha$-(1-S-Carboxy-3-cyclohexyl-propyl)-$N^\varepsilon$-benzyloxycarbonyl-S-lysyl-2S,3aS,7aS-octahydroindol-2-carbonsäurebenzylester

a) $N^\alpha$-tert. Butoxycarbonyl-$N^\varepsilon$-benzyloxycarbonyl-S-lysyl-2S,3aS,7aS-octahydroindol-2-carbonsäure-benzylester

Aus 2,8 mmol 2S,3aS,7aS-Octahydroindol-2-carbonsäurebenzylester wird mit NaOH die Base freigesetzt und mit 2,7 mmol $N^\alpha$-tert.Butoxycarbonyl-$N^\varepsilon$-benzyloxycarbonyl-S-lysin, 2,7 mmol 1-Hydroxybenztriazol und 2,7 mmol

N-Ethylmorpholin in absolutem DMF (20 ml) gelöst. Bei 0°C werden 2,7 mmol Dicyclohexylcarbodiimid zugesetzt, 2,5 Stunden bei Raumtemperatur gerührt, mit Essigester verdünnt, filtriert und das Filtrat je einmal mit 10 %iger Citronensäurelösung, 1 N Natriumbicarbonatlösung, Wasser und gesättigter Kochsalzlösung gewaschen, über Natriumsulfat getrocknet und eingeengt. Das Rohprodukt wird durch Filtration über eine kurze Kieselgelsäule mit Essigester/Cyclohexan (1 : 2) als Laufmittel gereinigt; farbloses viskoses Öl.

$^1$H-NMR ($CDCl_3$) : $\delta$ = 7,3 + 7,2 (2s, 10H);
5,0 (2s, 4H);
5,0 - 3,6 (m, 3H);
3,6 - 3,0 (m, 2H);
2,5 - 0,9 (m, 17H);
1,43 (s, 9H) ppm.

b) $N^\alpha$-Benzyloxycarbonyl-S-lysyl-2S,3aS,7aS-octahydroindol-2-carbonsäurebenzylester

1,4 g der Verbindung aus Beispiel 2 a) werden mit 10 ml Trifluoressigsäure 3 Stunden bei Raumtemperatur gerührt. Die überschüssige Säure wird mit Toluol im Vakuum azeotrop entfernt, der Rückstand in Wasser/Methanol aufgenommen und unter Rühren mittels Amberlite® IRA 93 (Acetat-Form) auf einen pH-Wert von 4,4 eingestellt, filtriert und eingeengt; 1,1 g farbloses Harz.

$^1$H-NMR ($CDCl_3$) : $\delta$ = 7,3 + 7,2 (2s, 10H);
5,1 (2s, 4H);
5,1 - 3,5 (m, 3H);
3,4 - 2,8 (m, 2H);
2,5 - 0,9 (m, 17H);

c) $N^{\alpha}$-(1-S-Carboxy-3-cyclohexyl-propyl)-N-benzyloxycarbonyl-S-lysyl-2S,3aS,7aS-octahydroindol-2-carbonsäurebenzylester

Eine Lösung von 2 g (7,1 mmol) 4-Cyclohexyl-2-trifluormethansulfonyloxybuttersäureethylester in 10 ml trockenem Methylenchlorid wird zu einer Mischung aus 7,0 mmol der Verbindung aus Beispiel 2 b) und 0,95 ml Triethylamin in 30 ml Methylenchlorid getropft. Nach 12 Stunden Rühren bei Raumtemperatur wird mit Wasser gewaschen mit Natriumsulfat getrocknet und eingeengt. Chromatographie wie bei 1 h) ergibt 3,2 g Produkt als farbloses Öl. Die $^{1}H$-NMR-Daten stimmen mit den für 1 h) angegebenen überein.

Patentansprüche

1. Verbindung der Formel I,

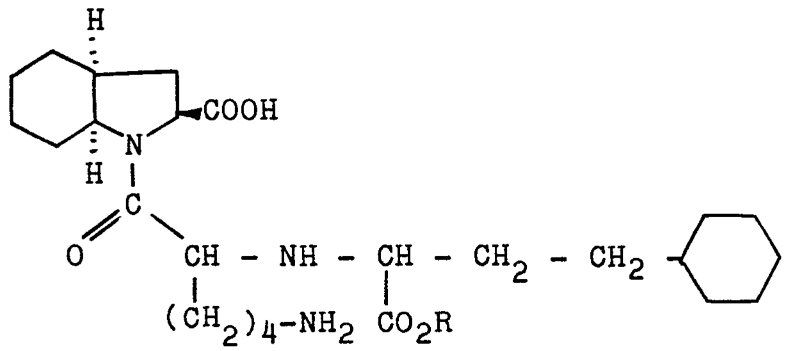

(I)

in welcher R Wasserstoff oder $(C_1-C_6)$-Alkyl bedeutet, und deren physiologisch verträglichen Salze.

2. Verbindung der Formel I gemäß Anspruch 1, dadurch gekennzeichnet, daß alle fünf Asymmetriezentren die S-Konfiguration aufweisen.

3. Verbindung der Formel I gemäß Anspruch 1 oder 2, in welcher R Ethyl bedeutet.

4. Verbindung der Formel I gemäß Anspruch 1 oder 2, in welcher R Wasserstoff bedeutet.

5. $N^{\alpha}$-(3-Cyclohexyl-1-S-ethoxycarbonyl)-propyl-S-lysyl-2S,3aS,7aS-octahydro[1H]indol-2-carbonsäure und deren physiologisch verträglichen Salze.

6. $N^{\alpha}$-(1-S-Carboxy-3-cyclohexyl)-propyl-S-lysyl-2S,3aS,7aS-octahydro[1H]indol-2-carbonsäure und deren physiologisch verträglichen Salze.

7. Verfahren zur Herstellung von Verbindungen der Formel I gemäß Anspruch I, dadurch gekennzeichnet, daß man

 

a) Verbindungen der Formel II,

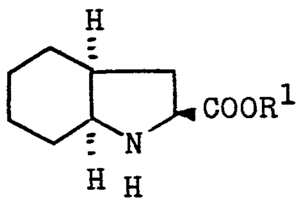

(II)

in welcher $R^1$ einen hydrogenolytisch oder hydrolytisch leicht abspaltbaren Rest bedeutet, mit Verbindungen der Formel III,

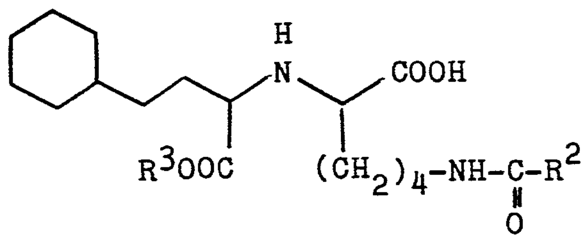

(III)

in welcher $R^2$ eine Urethan-Schutzgruppe und $R^3$ Benzyl oder $(C_1-C_6)$-Alkyl bedeutet, nach einer der üblichen Methoden der Peptidchemie umsetzt,

b) Verbindungen der Formel IV,

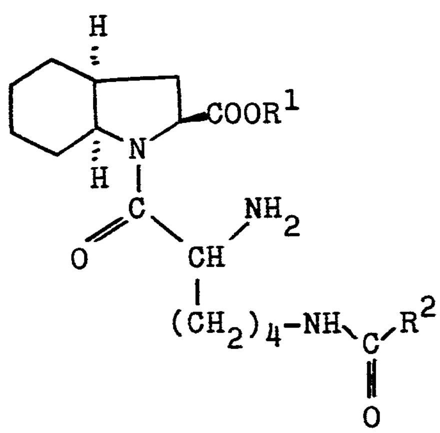

(IV)

in welcher $R^1$ und $R^2$ wie oben bei Formeln II und III definiert sind, mit Verbindungen der Formel V,

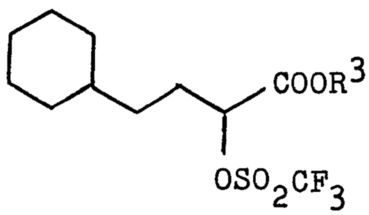

(V)

in welcher $R^3$ wie oben bei Formel III definiert ist, in Gegenwart einer Base umsetzt,

aus gegebenenfalls gebildeten Isomergemischen die gewünschten Isomeren abtrennt, die Schutzgruppen $R^1$ und $R^2$ abspaltet und $R^3$ gegebenenfalls abspaltet, was in einem Schritt oder in mehreren Schritten erfolgen kann, erhaltene Verbindungen der Formel I gegebenenfalls verestert und gegebenenfalls in physiologisch verträgliche Salze überführt, wobei bei einer Schutzgruppenabspaltung in mehreren Schritten die Veresterung auch auf einer früheren Verfahrensstufe erfolgen kann.

8. Verbindung gemäß einem der Ansprüche 1 bis 6, zur Anwendung als Heilmittel.

9. Mittel enthaltend eine Verbindung gemäß einem der Ansprüche 1 bis 6 und einen physiologisch verträglichen Träger.

10. Verwendung einer Verbindung gemäß einem der Ansprüche 1 bis 6 oder eines Mittels gemäß Anspruch 9 als Heilmittel.

Patentansprüche für den Vertragsstaat AT

1. Verfahren zur Herstellung von Verbindungen der Formel I

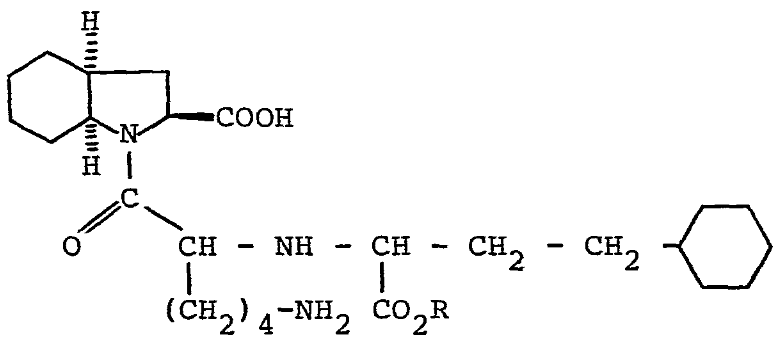

(I)

in welcher R Wasserstoff oder $(C_1-C_6)$-Alkyl bedeutet, und deren physiologisch verträglichen Salzen, dadurch gekennzeichnet, daß man

a) Verbindungen der Formel II,

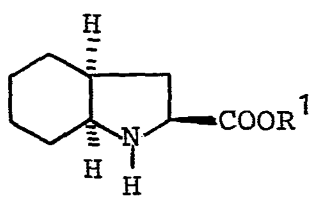

(II)

in welcher $R^1$ einen hydrogenolytisch oder hydrolytisch leicht abspaltbaren Rest bedeutet, mit Verbindungen der Formel III,

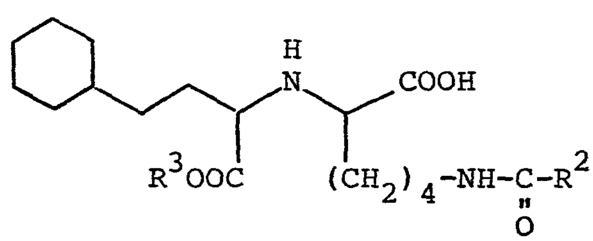

(III)

in welcher $R^2$ eine Urethan-Schutzgruppe und $R^3$ Benzyl oder $(C_1-C_6)$-Alkyl bedeutet, nach einer der üblichen Methoden der Peptidchemie umsetzt,

b) Verbindungen der Formel IV,

HOE 84/F 047 0154886

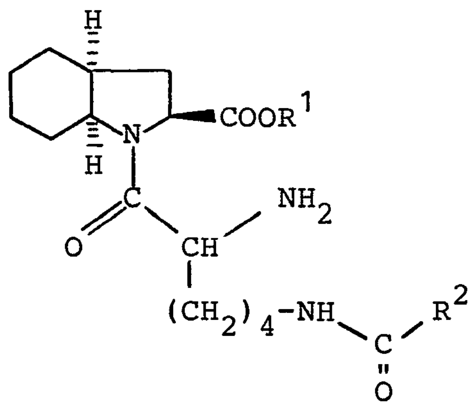

(IV)

in welcher $R^1$ und $R^2$ wie oben bei Formel II und III definiert sind, mit Verbindungen der Formel V,

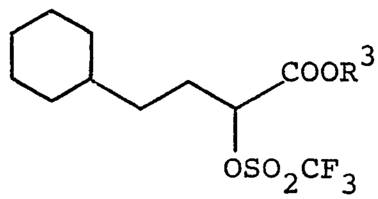

(V)

in welcher $R^3$ wie oben bei Formel III definiert ist, in Gegenwart einer Base umsetzt,

aus gegebenenfalls gebildeten Isomergemischen die gewünschten Isomeren abtrennt, die Schutzgruppen $R^1$ und $R^2$ abspaltet und $R^3$ gegebenenfalls abspaltet, was in einem Schritt oder in mehreren Schritten erfolgen kann, erhaltene Verbindungen der Formel I gegebenenfalls verestert und gegebenenfalls in physiologisch verträgliche Salze überführt, wobei bei einer Schutzgruppenabspaltung in mehreren Schritten die Veresterung auch auf einer früheren Verfahrensstufe erfolgen kann.

2. Verfahren gemäß Anspruch 1 zur Herstellung einer Verbindung der Formel I, worin alle fünf Asymmetriezentren die S-Konfiguration aufweisen.

3. Verfahren gemäß Anspruch 1 oder 2 zur Herstellung einer Verbindung der Formel I, worin R Ethyl bedeutet.

4. Verfahren gemäß Anspruch 1 oder 2 zur Herstellung einer Verbindung der Formel I, worin R Wasserstoff bedeutet.

5. Verfahren gemäß Anspruch 1 zur Herstellung von $N^{\alpha}$-(3-Cyclohexyl-1-S-ethoxycarbonyl)-propyl-S-lysyl-2S,3aS,7aS-octahydro/1H/indol-2-carbonsäure und deren physiologisch verträglichen Salzen.

6. Verfahren gemäß Anspruch 1 zur Herstellung von $N^{\alpha}$-(1-S-Carboxy-3-cyclohexyl)-propyl-S-lysyl-2S,3aS,7aS-octahydro/1H/indol-2-carbonsäure und deren physiologisch verträglichen Salzen.

7. Verfahren zur Herstellung eines Mittels enthaltend eine Verbindung gemäß einem der Ansprüche 1 bis 6 und einen physiologisch verträglichen Träger, dadurch gekennzeichnet, daß man diese in eine geeignete Darreichungsform bringt.